# EUROPÄISCHE PATENTANMELDUNG

(11) **EP 2 630 977 A2**
(43) Veröffentlichungstag der Anmeldung: **28.08.2013**
(21) Anmeldenummer: 12008511.3
(22) Anmeldetag: 21.12.2012
(51) Int. Cl.: A61L 27/16, A61L 24/06

(54) **Pastenförmiger Knochenzement**

(30) Priorität: 30.01.2012 DE 102012001636
(71) Anmelder: Heraeus Medical GmbH, 61273 Wehrheim (DE)
(72) Erfinder: Vogt, Sebastian, 99092 Erfurt (DE)
(74) Vertreter: Panten, Kirsten

(57) **Zusammenfassung**

Die vorliegende Erfindung betrifft eine Paste, beinhaltend wenigstens ein radikalisch polymerisierbares Monomer, wenigstens ein in dem wenigstens einen radikalisch polymerisierbaren Monomer lösliches Polymer und wenigstens einen in dem wenigstens einen radikalisch polymerisierbaren Monomer schwer- oder unlöslichen Füllstoff, wobei der Füllstoff ein partikuläres, vernetztes Polymethacrylat ist, welches durch Polymerisation von Methacrylsäureestem herstellbar ist, wobei
i) mindestens 15 Gew.-% der zur Polymerisation eingesetzten Methacrylsäureester mehrfachfunktionelle Methacrylsäureester sind, bezogen auf das Gesamtgewicht der bei Polymerisation eingesetzten Methacrylsäureester, und
ii) mindestens 90 Gew.-% der Partikel des Füllstoffes, bezogen auf das Gesamtgewicht des Füllstoffes, eine Partikelgröße von nicht mehr als 74 µm aufweisen.

Die Erfindung betrifft auch einen Kit, die Verwendung eines in einem radikalisch polymerisierbaren Monomer schwer- oder unlöslichen Füllstoffs sowie die Verwendung einer Paste oder einer Paste, hergestellt aus dem erfindungsgemäßen Kit, zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie.

## Beschreibung

Die vorliegende Erfindung betrifft eine Paste, einen Kit, die Verwendung eines in einem radikalisch polymerisierbaren Monomer schwer- oder unlöslichen Füllstoffs sowie die Verwendung einer Paste oder einer Paste, hergestellt aus einem Kit, zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie.

Konventionelle Polymethylmethacrylat-Knochenzemente (PMMA-Knochenze-mente) sind seit Jahrzehnten bekannt und gehen auf die grundlegenden Arbeiten von Sir Charnley zurück (Charnley, J.: "Anchorage of the femoral head prosthesis of the shaft of the femur"; J, Bone Joint Surg. 42 (1960) 28-30). Der Grundaufbau der PMMA-Knochenzemente ist seitdem prinzipiell gleich geblieben. PMMA-Knochenzemente bestehen aus einer flüssigen Monomerkomponente und einer Pulverkomponente. Die Monomerkomponente enthält im Allgemeinen (i) das Monomer Methylmethacrylat und (ii) einen darin gelösten Aktivator (zum Beispiel N,N-Dimethyl-p-toluidin). Die Pulverkomponente umfasst (i) ein oder mehrere Polymere, die auf Basis von Methylmethacrylat und Comonomeren, wie Styrol, Methylacrylat oder ähnlichen Monomeren, durch Polymerisation, vorzugsweise durch Suspensionspolymerisation, hergestellt sind, (ii) einen Röntgenopaker und (iii) einen Initiator (zum Beispiel) Dibenzoylperoxid. Beim Vermischen der Pulverkomponente mit der Monomerkomponente entsteht durch Quellung der Polymere der Pulverkomponente im Methylmethacrylat der Monomerkomponente ein plastisch verformbarer Teig. Gleichzeitig reagiert der Aktivator N,N-Dimethyl-p-toluidin mit Dibenzoylperoxid, das unter Bildung von Radikalen zerfällt. Die gebildeten Radikale initiieren die radikalische Polymerisation des Methylmethacrylates. Mit fortschreitender Polymerisation des Methylmethacrylates erhöht sich die Viskosität des Zementteigs bis der Zementteig erstarrt und damit ausgehärtet ist

Als Alternative zu den konventionellen Pulver-Flüssigkeits-Polymethylmeth-acrylatknochenzementen wurden pastenförmige Polymethylmethacrylatknochenzemente in den Offenlegungsschriften DE-A-10 2007 052 116, DE-A-10 2007 050 762 und DE-A-10 2007 050 763 beschrieben, welche ein radikalisch polymerisierbares Methacrylat-Monomer, ein in diesem Methacrylat-Monomer lösliches Polymer und gegebenenfalls ein in diesem Methacrylat-Monomer unlösliches partikuläres Polymer beinhalten. Solche pastenförmigen Polymethylmethacrylat-Knochenzemente können als Einkomponenten-System (in diesem Fall beinhaltet die Paste alle zur Aushärtung notwendigen Komponenten, insbesondere einen aktivierbaren radikalischen Initiator, z. B. einen Photoinitiator oder ein Photoiniatorsystem) oder als Zweikomponenten-System (in diesem Fall umfasst das System zwei vorgemischte, lagerstabile Pasten, von denen eine einen radikalischen Polymerisationsinitiator und die andere einen Polymerisationsaktivator aufweist) vorliegen. Im Falle der Zweikomponenten-Systeme wird weiterhin zwischen einem "symmetrischen System" (in diesem Fall beinhalten beide Pasten ein in dem Methacrylat-Monomer unlösliches partikuläres Polymer) und "unsymmetrischen Systemen" (in diesem Fall beinhaltet nur eine der beiden Pasten ein in dem Methacrylat-Monomer unlösliches partikuläres Polymer) unterschieden.

Der aus den vorstehen beschriebenen Pasten hergestellte Knochenzement weist als Folge der gewählten Zusammensetzung eine ausreichend hohe Viskosität und Kohäsion auf, um dem Blutungsdruck bis zur Aushärtung standzuhalten. Durch die fortschreitende Polymerisation härtet die Paste unter Verbrauch der Methacrylat-Monomere aus.

In Methacrylat-Monomeren unlösliche anorganische Füllstoffe führen zu einer ausgeprägten Sprödigkeit der ausgehärteten Pastenzemente, die häufig zu Brüchen im Korn führt. Bei der Verwendung von in Methacrylat-Monomeren quellbaren organischen, partikulären Füllstoffen wiederum zeigte sich, dass bei ausgeprägter Aufnahme der Methacrylat-Monomere nicht sofort unmittelbar nach der Aushärtung der Zementpasten, sondern erst verzögert die Endfestigkeit des ausgehärteten Zementes erreicht wird. Bei allen bisher bekannten, auf Zementpulver und Monomerflüssigkeit aufgebauten Polymethylmethacrylat-Knochenzementen verbleibt infolge des Einfrierens der Polymerisation immer ein gewisser Restmonomergehalt, der erst im Laufe von Stunden bis Tagen nachpolymerisiert. Bei der Aushärtung von pastenförmigen Knochenzementen zeigte sich jedoch eine etwas ausgeprägte Nachhärtung. Dies ist darauf zurück zu führen, dass das in den gequollenen Polymerpartikeln durch Quellung aufgenommene Methacrylat-Monomer nur teilweise während der Aushärtung der die gequollenen Partikel umgebenden Zementmatrix an der Polymerisation teilnimmt. Das bedeutet, das nicht polymerisierte Methacrylat-Monomer kann nach der Aushärtung der Zementmatrix aus den Partikeln heraus diffundieren und bis zu seiner Nachpolymerisation temporär als Weichmacher wirken. Andererseits zeigten Versuche, dass organische Füllstoffe, die keine oder nur minimale Mengen an Meacrylat-Monomer aufnehmen, nur sehr schlecht an die Zementmatrix angebunden werden und damit den ausgehärteten Zement mechanisch schwächen.

Der vorliegenden Erfindung lag die Aufgabe zugrunde, die sich aus dem Stand der Technik ergebenden Nachteile im Zusammenhang mit auf Pasten basierenden Knochenzement-Systemen, insbesondere im Zusammenhang mit den vorstehend beschriebenen Einkomponenten-Systemen und Zweikomponenten-Systemen, zu überwinden.

Auch lag der vorliegenden Erfindung die Aufgabe zugrunde, eine Knochenzementpaste bereitzustellen, die eine möglichst geringe Neigung zur Nachhärtung aufweist und die zugleich nach Aushärtung zu einem Knochenzement führt, der eine möglichst vorteilhafte mechanische Stabilität, insbesondere eine möglichst geringe Sprödigkeit, aufweist.

Gelöst wurde diese Aufgabe dadurch, dass in herkömmlichen Einkomponenten- oder Zweikomponenten-Systemen als in einem Methacrylat-Monomer unlöslicher partikulärer Füllstoff ein partikuläres, vernetztes Polymethacrylat eingesetzt wird, welches nur geringe Mengen an Methacrylat-Monomer durch Quellung aufnimmt und andererseits eine gute Verbindung mit der Pastenmatrix ausbildet, die bei der Aushärtung des Knochenzements durch Polymerisation des Methacrylat-Monomers in Gegenwart eines in dem Methacrylat-Monomer löslichen Polymers erhaltenen wird.

Einen Beitrag zur Lösung der eingangs genannten Aufgaben leistet eine Paste, beinhaltend wenigstens ein radikalisch polymerisierbares Monomer, wenigstens ein in dem wenigstens einen radikalisch polymerisierbaren Monomer lösliches Polymer und wenigstens einen in dem wenigstens einen radikalisch polymerisierbaren Monomer schwer- oder unlöslichen Füllstoff, wobei der Füllstoff ein partikuläres, vernetztes Polymethacrylat ist, welches durch Polymerisation von Methacrylsäureestem herstellbar ist, vorzugsweise hergestellt worden ist, wobei
i) mindestens 15 Gew.-% der zur Polymerisation eingesetzten Methacrylsäureester mehrfachfunktionelle Methacrylsäureester sind, bezogen auf das Gesamtgewicht der bei Polymerisation (gemeint ist hier die Polymerisation, die zur Herstellung der partikulären, vernetzten Polymethacrylate führt, nicht aber die Polymerisation, die bei der Aushärtung der Paste erfolgt) eingesetzten Methacrylsäureester, und
ii) mindestens 90 Gew.-% der Partikel des Füllstoffes, bezogen auf das Gesamtgewicht des Füllstoffes, eine Partikelgröße von nicht mehr als 74 µm aufweisen.

Grundsätzlich kann die erfindungsgemäße Paste ein vorstehend beschriebenes Einkomponenten-System sein oder durch Vermischen der beiden Pasten eines vorstehend beschriebenen Zweikomponenten-Systems erhalten werden.

Die erfindungsgemäße Paste beinhaltet als eine Komponente wenigstens ein radikalisch polymerisierbares Monomer, wobei es sich vorzugsweise um ein Methacrylat-Monomer, insbesondere um ein Methacrylat-Monomer handelt, welches bei einer Temperatur von 25°C und einem Druck von 1.013 hPa flüssig ist.

Das Methacrylat-Monomer ist vorzugsweise ein Methacrylsäureester. Vorzugsweise handelt es sich bei dem Methacrylsäureester um einen monofunktionellen Methacrylsäureester. Dieser ist vorzugsweise hydrophob. Durch die Verwendung von hydrophoben monofunktionellen Methacrylsäureestem kann eine spätere Volumenvergrößerung des Knochenzements infolge von Wasseraufnahme und damit eine Beschädigung des Knochens verhindert werden. Gemäß einer bevorzugten Ausführungsform ist der monofunktionelle Methacrylsäureester hydrophob, wenn er neben der Estergruppe keine weiteren polaren Gruppen enthält. Vorzugsweise weist der monofunktionelle, hydrophobe Methacrylsäureester keine Carboxylgruppen, Hydroxylgruppen, Amidgruppen, Sulfonsäuregruppen, Sulfatgruppen, Phosphatgruppen oder Phosphonatgruppen auf.

Bei den Estern handelt es sich vorzugsweise um Alkylester. Unter Alkylester fallen erfindungsgemäß auch Cycloalkylester. Die Alkylester sind gemäß einer bevorzugten Ausführungsform Ester von Methacrylsäure mit Alkoholen, die 1 bis 20 Kohlenstoffatome, mehr bevorzugt 1 bis 10 Kohlenstoffatome, noch mehr bevorzugt 1 bis 6 Kohlenstoffatome und ganz besonders bevorzugt 1 bis 4 Kohlenstoffatome aufweisen. Die Alkohole können substituiert oder nicht substituiert sein und sind vorzugsweise nicht substituiert. Die Alkohole können ferner gesättigt oder ungesättigt sein und sind vorzugsweise gesättigt.

Das erfindungsgemäß eingesetzte radikalisch polymerisierbare Monomer weist vorzugsweise eine Molmasse von weniger als 1.000 g/mol auf. Davon umfasst sind auch radikalisch polymerisierbare Monomere, die Bestandteil einer Monomermischung sind, wobei wenigstens eines der radikalisch polymerisierbaren Monomere der Monomermischung eine definierte Struktur mit einer Molmasse von weniger als 1.000 g/mol aufweist.

Das radikalisch polymerisierbare Monomer zeichnet sich vorzugsweise dadurch aus, dass eine wässrige Lösung des radikalisch polymerisierbaren Monomers einen pH-Wert im Bereich von 5 bis 9, vorzugsweise im Bereich von 5,5 bis 8,5, noch mehr bevorzugt im Bereich von 6 bis 8 und ganz besonders bevorzugt im Bereich von 6,5 bis 7,5 aufweist.

Gemäß einer besonders bevorzugten Ausführungsform handelt es sich bei dem Methacrylat-Monomer um Methacrylsäuremethylester, Methacrylsäureethylester oder um eine Mischung aus diesen beiden Monomeren.

Vorzugsweise beinhaltet die erfindungsgemäße Paste das radikalisch polymerisierbare Monomer in einer Menge in einem Bereich von 15 bis 85 Gew.-%, mehr bevorzugt 20 bis 70 Gew.-%, noch mehr bevorzugt 25 bis 60 Gew,-% und besonders bevorzugt 25 bis 50 Gew.-%, jeweils bezogen auf das Gesamtgewicht der erfindungsgemäßen Paste.

Als weitere Komponente beinhaltet die erfindungsgemäße Paste wenigstens ein in dem wenigstens einen radikalisch polymerisierbaren Monomer lösliches Polymer. Ein Polymer ist erfindungsgemäß in dem polymerisierbaren Monomer löslich, wenn sich das Polymer in diesem polymerisierbaren Monomer zu wenigstens 10 g/l, vorzugsweise zu wenigstens 25 g/l, mehr bevorzugt zu wenigstens 50 g/l und ganz besonders bevorzugt zu wenigstens 100 g/l, löst. Das in dem polymerisierbaren Monomer lösliche Polymer kann ein Homopolymer oder ein Copolymer sein. Bei diesem löslichen Polymer handelt es sich vorzugsweise um ein Polymer mit einer gewichtsmittleren Molmasse von wenigstens 150,000 g/mol. Beispielsweise kann es sich bei dem löslichen Polymer um ein Polymer oder Copolymer eines Methacrylsäureesters handeln. Gemäß einer besonders bevorzugten Ausführungsform ist das wenigstens eine lösliche Polymer aus der Gruppe ausgewählt, die aus Polymethacrylsäuremethylester (PMMA), Polymethacrylsäureethylester (PMAE), Polymethacrylsäurepropylester (PMAP), Polymethacrylsäureisopropylester, Poly(methylmethacrylat-co-methylacrylat), Poly(styren-co-methylmeth-acrylat) und einer Mischung aus mindestens zwei dieser Polymere besteht.

Die Menge des in dem radikalisch polymerisierbaren Monomer löslichen Polymers in der erfindungsgemäßen Paste liegt üblicherweise in einem Bereich von 1 bis 85 Gew.-%, bezogen auf das Gesamtgewicht der erfindungsgemäßen Paste.

Weiterhin beinhaltet die erfindungsgemäße Paste wenigstens einen in dem wenigstens einen radikalisch polymerisierbaren Monomer schwer- oder unlöslichen Füllstoff, wobei der Füllstoff ein partikuläres, vernetztes Polymethacrylat ist, welches durch Polymerisation von Methacrylsäureestem hergestellt worden ist, wobei
i) mindestens 15 Gew.-% der zur Polymerisation eingesetzten Methacrylsäureester mehrfachfunktionelle Methacrylsäureester sind, bezogen auf das Gesamtgewicht der bei Polymerisation eingesetzten Methacrylsäureester, und
ii) mindestens 90 Gew.-% der Partikel des Füllstoffes, bezogen auf das Gesamtgewicht des Füllstoffes, eine Partikelgröße von nicht mehr als 74 µm aufweisen.

Bei dem schwer- oder unlöslichen Füllstoff handelt es sich um einen bei Raumtemperatur festen Stoff, der in der Lage ist, die Viskosität der aus den übrigen in der erfindungsgemäßen Paste enthaltenen Bestandteilen zusammengesetzten Mischung zu erhöhen. Der Füllstoff soll biokompatibel sein.

Der Füllstoff ist in dem radikalisch polymerisierbaren Monomer schwer- oder unlöslich. Erfindungsgemäß ist der Füllstoff in dem radikalisch polymerisierbaren Monomer schwer- oder unlöslich, wenn der Füllstoff bei einer Temperatur von 25°C eine Löslichkeit im radikalisch polymerisierbaren Monomer von weniger als 50 g/l, vorzugsweise weniger als 25 g/l, mehr bevorzugt weniger als 10 g/l, noch mehr bevorzugt weniger als 5 g/l und noch mehr bevorzugt weniger als 0,5 g/l aufweist, wobei es am meisten bevorzugt ist, dass sich der schwer- oder unlösliche Füllstoff in dem radikalisch polymerisierbaren Monomer überhaupt nicht oder aber allenfalls in vernachlässigbar geringer Menge löst.

Weiterhin ist der Füllstoff partikulär, wobei mindestens 90 Gew.-%, besonders bevorzugt mindestens 95 Gew.-% und am meisten bevorzugt 100 Gew.-% der Partikel des Füllstoffes, jeweils bezogen auf das Gesamtgewicht des Füllstoffes, eine Partikelgröße von nicht mehr als 74 µm, besonders bevorzugt von nicht mehr als 63 µm und am meisten bevorzugt von nicht mehr als 53 µm aufweisen. Die Formulierung *"wobei mindestens 90 Gew.-% der Partikel des Füllstoffes, bezogen auf das Gesamtgewicht des Füllstoffes, eine Partikelgröße von nicht mehr als 74* µ*m aufweisen"* soll ausdrücken, dass beim Absieben des Füllstoffes über ein Sieb mit einer Maschenweite von 74 µm (mesh-200-Sieb) maximal 10 Gew.-% der Partikel auf dem Sieb verbleiben. Dementsprechend ist mit der Formulierung *"wobei mindestens 90 Gew.-% der Partikel des Füllstoffes, bezogen auf das Gesamtgewicht des Füllstoffes, eine Partikelgröße von nicht mehr als 63 µm aufweisen"* gemeint, dass beim Absieben des Füllstoffes über ein Sieb mit einer Maschenweite von 63 µm (mesh-230-Sieb) maximal 10 Gew.-% der Partikel auf dem Sieb verbleiben und mit der mit der Formulierung *"wobei mindestens 90 Gew.-% der Partikel des Füllstoffes, bezogen auf das Gesamtgewicht des Füllstoffes, eine Partikelgröße von nicht mehr als 53* µ*m aufweisen",* dass beim Absieben des Füllstoffes über ein Sieb mit einer Maschenweite von 53 µm (mesh-270-Sieb) maximal 10 Gew.-% der Partikel auf dem Sieb verbleiben.

Der partikuläre Füllstoff ist ein vernetztes Polymethacrylat, welches durch Polymerisation von Methacrylsäureestern herstellbar ist, vorzugsweise hergestellt worden ist, wobei mindestens 15 Gew.-%, besonders bevorzugt mindestens 20 Gew.-% der zur Polymerisation eingesetzten Methacrylsäureester mehrfachfunktionelle Methacrylsäureester sind, jeweils bezogen auf das Gesamtgewicht der bei Polymerisation eingesetzten Methacrylsäureester. Vorzugsweise weist das vernetzte Polymethylmethacrylat eine gewichtsmittlere Molmasse von wenigstens 150.000 g/mol auf. -Die Angabe der Molmasse bezieht sich auf die durch Gelpermeationschromatopgaphie (GPC) bestimmte gewichtsmittlere Molmasse.

Gemäß einer besonderen Ausgestaltung der erfindungsgemäßen Paste beinhaltet diese zwei verschiedene, die Merkmale i) und ii) verwirklichende partikulare, vernetzte Polymethacrylate P1 und P2, wobei
- P1 durch Polymerisation von 90 bis 100 Gew.-% mehrfachfunktionellen Methacrylsäureestern und 0 bis 10 Gew.-% monofunktionellen Methacrylsäureestem, jeweils bezogen auf das Gesamtgewicht der bei Polymerisation eingesetzten Methacrylsäureester und
- P2 durch Polymerisation von 20 bis 40 Gew.% mehrfachfunktionellen Methacrylsäureestern und 60 bis 80 Gew.-% monofunktionellen Methacrylsäureestern, jeweils bezogen auf das Gesamtgewicht der bei Polymerisation eingesetzten Methacrylsäureester.

Bei dem mehrfachfunktionalisierten Methacrylsäureester handelt es sich vorzugsweise um einen mehrfachfunktionalisierten Methacrylsäureester ausgewählt aus der Gruppe bestehend aus Ethylenglykoldimethacrylat, Butan-1,4-diol-dimethacrylat, Hexan-1,6-dioldimethacrylat, Dodecan-1,12-diol-dimethacrylat, Diethylenglykoldimethacrylat, Trimethylenglykoltrimethacrylat, Glycerin-1,2,3-trimethacrylat und Pentaerythroltetramethacrylat. Sofern weitere Co-Monomere zur Herstellung der partikulären, vernetzten Polymethacrylate eingesetzt werden, so handelt es sich bei diesen Co-Monomeren vorzugsweise um monofunktionelle Methacrylsäureester, besonders bevorzugt um Methylmethacrylat.

Das partikuläre, vernetzte Polymethacrylat kann kugelförmig oder auch asphärisch geformt sein. Annähernd kugelförmiges vernetztes Polymethylmethacrylat wird durch Suspensionspolymerisation hergestellt. Das asphärisch geformte, partikuläre, vernetzte Polymethacrylat wird durch thermisch induzierte Massepolymerisation von Methacrylsäureestern hergestellt und nach der Polymerisation durch Aufmahlung des Polymerisats bis auf die Wunschkomgröße zerkleinert. Das zerkleinerte Polymerisat wird als Splitterpolymerisat bezeichnet.

Gegebenenfalls kann die erfindungsgemäße Paste neben dem vorstehend beschriebenen partikulären, vernetzten Polymethacrylat auch weitere Füllstoffe beinhalten, wie etwa anorganische Salze, anorganische Oxide, Metalle oder Metalllegierungen.

Vorzugsweise handelt es sich bei dem anorganischen Salz um ein Salz eines Elements, das aus der 2. Hauptgruppe des Periodensystems der Elemente ausgewählt wird. Gemäß einer bevorzugten Ausführungsform ist das anorganische Salz ein Salz von Calcium, Strontium oder Barium. Gemäß einer besonders bevorzugten Ausführungsform ist das anorganische Salz Kalziumsulfat, Bariumsulfat oder Kalziumcarbonat. Bei dem als Füllstoff verwendbaren anorganischen Oxid kann es sich vorzugsweise um ein Metalloxid handeln. Gemäß einer bevorzugten Ausführungsform ist das anorganische Oxid ein Oxid der Übergangsmetalle. Gemäß einer besonders bevorzugten Ausführungsform handelt es sich bei dem anorganischen Oxid um Titandioxid oder Zirkoniumdioxid. Bei dem als Füllstoff verwendbaren Metall kann es sich beispielsweise um ein Übergangsmetall handeln. Gemäß einer bevorzugten Ausführungsform ist das Metall Tantal oder Wolfram. Die als Füllstoff verwendbare Metalllegierung ist eine Legierung von wenigstens zwei Metallen. Vorzugsweise enthält die Legierung wenigstens ein Übergangsmetall. Gemäß einer besonders bevorzugten Ausführungsform weist die Legierung wenigstens Tantal oder Wolfram auf. Bei der Legierung kann es sich auch um eine Legierung aus Tantal und Wolfram handeln.

Die Menge an Füllstoff, besonders bevorzugt die Menge an dem vorstehend beschriebenen partikulären, vernetzten Polymethacrylat in der erfindungsgemäßen Paste beträgt vorzugsweise 15 bis 85 Gew.-%, besonders bevorzugt 15 bis 80 Gew.-% und noch mehr bevorzugt 20 bis 75 Gew.-%, jeweils bezogen auf das Gesamtgewicht der erfindungsgemäßen Paste.

Die erfindungsgemäße Paste kann weiterhin wenigstens einen Polymerisationsinitiator, wenigstens einen Polymerisationsbeschleuniger oder wenigstens einen Polymerisations-initiator und einen Polymerisationsbeschleuniger beinhalten.

Im Falle eines Einkomponenten-Systems handelt es sich bei dem Polymerisationsinitiator vorzugsweise um einen aktivierbaren Polymerisationsinitiator, z. B. um einen in der Paste gelösten oder suspendierten Photoinitiator oder um ein in der Paste gelöstes oder suspendiertes Photoiniatorsystem. Es ist auch möglich, einen Initiator oder Initiatoren dort vorzusehen, wo er bzw. sie vorübergehend zu Kontakt mit der Paste kommt, etwa in einem Behälterteil, einer Dosiervorrichtung oder einer Transportkanüle. Auch kann im Falle eines Einkomponenten-Systems die erfindungsgemäße Paste neben dem aktivierbaren Polymerisationsinitiator auch einen elektrisch leitfähigen Röntgenopaker beinhalten. Hier eignen sich besonders Partikel von Kobalt, Eisen, NdFeB, SmCo, Kobalt-Chrom-Stahl, Zirkonium, Hafnium, Titan, Titan-Aluminium-Silizium-Legierungen und Titan-Niobium-Legierungen mit einer Partikelgröße von 0,5-500 µm. In dem elektrisch leitfähigen Röntgenopaker können durch magnetische Wechselfelder mit einer Frequenz im Bereich von 500 Hz bis 50 kHz Wirbelströme induziert werden, die zu einer Erwärmung des Opakers führen. Durch Wärmeübertragung wird auch der Initiator aufgeheizt und zum thermischen Zerfall gebracht.

Im Falle einer erfindungsgemäßen Paste, welche durch die Kombination zweier Pasten eines Zweikomponenten-Systems erhalten wurde, beinhaltet diese Paste vorzugsweise wenigstens einen Polymerisationsinitiator (der in der einen Paste des Zweikomponenten-Systems enthalten war) und wenigstens einen Polymerisationsbeschleuniger (der in der anderen Paste des Zweikomponenten-Systems enthalten war).

Als Polymerisationsinitiator kommen insbesondere Peroxide und Barbitursäurederivate in Betracht, wobei die Peroxide und Barbitursäurederivate vorzugsweise in dem polymerisierbaren Monomer zu wenigstens 1 g/l, vorzugsweise zu wenigstens 3 g/l, noch mehr bevorzugt zu wenigstens 5 g/l und ganz besonders bevorzugt zu wenigstens 10 g/l bei einer Temperatur von 25°C löslich sind.

Unter einem Peroxid werden erfindungsgemäß Verbindungen verstanden, die wenigstens eine Peroxogruppe (-O-O-)enthalten. Das Peroxid weist vorzugsweise keine freien aciden Gruppen auf. Bei dem Peroxid kann es sich um ein anorganisches Peroxid, wie zum Beispiel ein toxikologisch unbedenkliches Hydroperoxid, oder ein organisches Peroxid handeln. Gemäß einer besonders bevorzugten Ausführungsform ist das Peroxid aus der Gruppe ausgewählt, die aus Dibenzoylperoxid und Dilauroylperoxid besteht.

Bei dem Barbitursäurederivat handelt es sich vorzugsweise um ein Barbitursäurederivat ausgewählt aus der Gruppe bestehend aus 1-monosubstituierten Barbituraten, 5-monosubstituierten Barbituraten, 1,5-disubstituierten Barbituraten-und 1,3,5-trisubstituierten Barbituraten -, Gemäß einer besonderen Ausgestaltung der erfindungsgemäßen Paste ist das Barbitursäurederivat ausgewählt ist aus der Gruppe bestehend aus 1,5-disubstituierten Barbituraten-und 1,3,5-trisubstituierten Barbituraten -,

Die Art der Substituenten an der Barbitursäure ist nicht weiter eingeschränkt. Bei den Substituenten kann es sich beispielsweise um aliphatische oder aromatische Substituenten handeln. Hierbei können alkylische, cycloalkylische, allylische oder arylische Substituenten bevorzugt sein. Die Substituenten können auch Heteroatome tragen. Insbesondere kann es sich bei den Substituenten um Thiosubstituenten handeln. Demnach können 1,5-disubstituierte Thiobarbiturate oder 1,3,5-trisubstituierte Thiobarbiturate bevorzugt sein. Gemäß einer bevorzugten Ausführungsform weisen die Substituenten jeweils eine Länge von 1 bis 10 Kohlenstoffatomen, mehr bevorzugt eine Länge von 1 bis 8 Kohlenstoffatomen und ganz besonders bevorzugt eine Länge im Bereich von 2 bis 7 Kohlenstoffatomen auf. Erfindungsgemäß bevorzugt sind Barbiturate, die an Position 1 und an Position 5 jeweils einen Substituenten oder an den Positionen 1, 3 und 5 jeweils einen Substituenten -tragen. Gemäß einer weiteren bevorzugten Ausführungsform ist das Barbitursäure-Derivat ein 1,5-disubstituiertes Barbiturat oder ein 1,3,5-trisubtituiertes Barbiturat. Gemäß einer besonders bevorzugten Ausführungsform ist das Barbitursäurederivat aus der Gruppe ausgewählt, die aus 1-Cyclohexyl-5-ethyl-barbitursäure, 1-Phenyl-5-ethyl-barbitursäure und 1,3,5-Trimethyl-barbitursäure besteht.

Als Polymerisationsbeschleuniger sind Schwermetallverbindungen, die aus der Gruppe bestehend aus Schwermetallsalzen und Schwermetallkomplexen ausgewählt sind, bevorzugt, wobei es sich als besonders vorteilhaft erwiesen hat, wenn die Schermetallverbindung in dem radikalisch polymerisierbaren Monomer schwer-, vorzugsweise sogar unlöslich ist. Als in dem radikalisch polymerisierbaren Monomer schwer- oder unlöslich gilt eine Schwermetallverbindung, wenn sich weniger als 1 g/l, vorzugsweise weniger als 0,1 g/l, noch mehr bevorzugt weniger als 0,01 g/l, noch mehr bevorzugt weniger als 0,001 g/l, darüber hinaus bevorzugt weniger als 0,0001 g/l um am meisten bevorzugt überhaupt keine nennenswerten Mengen der Schwermetallverbindung bei einer Temperatur von 25°C in dem radikalisch polymerisierbaren Monomer lösen (die Schwermetallverbindung also in dem radikalisch polymerisierbaren Monomer unlöslich ist). Unter Schwermetallverbindungen werden erfindungsgemäß Verbindungen von Metallen verstanden, die eine Dichte bei einer Temperatur von 20°C von wenigstens 3,5, vorzugsweise von wenigstens 5 aufweisen. Gemäß einer bevorzugten Ausführungsform handelt es sich bei der Schwermetallverbindung um eine basische Schwermetallverbindung. Unter basischer Schwermetallverbindung wird eine Schwermetallverbindung verstanden, die in Wasser gelöst oder suspendiert einen pH-Wert von wenigstens 7,0, bevorzugt wenigstens 8 und noch mehr bevorzugt wenigstens 8,5 aufweist. Gemäß einer ganz besonders bevorzugten Ausführungsform handelt es sich bei den Schwermetallverbindungen um Verbindungen von Metallen, die ihre Oxidationsstufe wechseln können. In diesem Zusammenhang erfindungsgemäß bevorzugt sind Verbindungen von Kupfer(II), Eisen(II), Eisen(III), Mangan(II), Mangan(III), Cobalt(II) und Cobalt(III), wobei Kupfer(II)-Verbindungen ganz besonders bevorzugt sind. Die erfindungsgemäßen Schwermetallverbindungen sind, sofern es sich um in dem radikalisch polymerisierbaren Monomer schwer- oder unlösliche Schermetallverbindungen handelt, vorzugsweise in der Lage, sich in Gegenwart der Barbitursäurederivate in eine in dem radikalisch polymerisierbaren Monomer lösliche Form umzuwandeln. Erfindungsgemäß handelt es sich bei den Schwermetallverbindungen vorzugsweise um Schwermetallsalze oder Schwermetallkomplexe. Bei den Schwernetallsalzen handelt es sich vorzugsweise um Halogenide, Hydroxyde, Carbonate oder Carbonsäuresalze von Schwermetallen. Bevorzugte Schwermetallsalze sind Salze von Kupfer(II), Eisen(II), Eisen(III), Mangan(II), Mangan(III), Cobalt(II) und Cobalt(III). Als Schwermetallverbindung kommen weiterhin Halogenidsalze in Betracht. Das Halogenidsalz kann vorzugsweise aus der Gruppe ausgewählt sein, die aus Chloriden und Bromiden von Schwermetallen besteht. Gemäß einer besonderen Ausführungsform handelt es sich bei dem Halogenidsalz um eine Verbindung, die aus der Gruppe ausgewählt ist, die aus Mangan(II)-chlorid, Eisen(II)chlorid, Eisen(III)-chlorid, Kobalt(II)-chlorid und Kobalt(III)-chlorid, besteht. Gemäß einer besonders bevorzugten Ausführungsform wird das Sohwermetallsalz aus der Gruppe ausgewählt, die aus Kupfer(II)hydroxid, basischem Kupfer(II)-carbonat oder einer Mischung aus mindestens zwei davon, insbesondere einer Mischung aus Kupfer(II)hydroxid und Kupfer(II)carbonat, besteht

Die erfindungsgemäße Paste kann den Polymerisationsinitiator, den Polymerisationsbeschleuniger oder den Polymerisationsinitiator und den Polymerisationsbeschleuniger in einer (Gesamt)Menge von bis zu 5 Gew.-%, bezogen auf das Gesamtgewicht der erfindungsgemäßen Paste, beinhalten.

Die erfindungsgemäße Paste kann neben den vorstehend genannten Komponenten weitere Bestandteile beinhalten.

Gemäß einer bevorzugten Ausführungsform der erfindungsgemäßen Paste kann diese wenigstens ein Röntgenopaker beinhalten. Beim Röntgenopaker kann es sich um einen fachüblichen Röntgenopaker handeln. Geeignete Röntgenopaker können in dem radikalisch polymerisierbaren Monomer löslich oder unlöslich sein. Der Röntgenopaker ist vorzugsweise aus der Gruppe ausgewählt, die aus Metalloxiden (wie zum Beispiel Zirkoniumdioxid), Bariumsulfat, toxikologisch unbedenklichen Schwermetallpartikeln (wie zum Beispiel Tantal), Ferrit, Magnetit (ggf. auch supramagnetisches Magnetit) und biokompatiblen Kalziumsalzen besteht. Diese Röntgenopaker weisen vorzugsweise einen mittleren Teilchendurchmesser im Bereich von 10 nm bis 500 µm auf. Als Röntgenopaker kommen ferner auch Ester der 3,5-Bis(acetamido)-2,4,6-triiodbenzoesäure, Gadolinium-Verbindungen, wie Gadolinium-Chelat mit den Estern der 1,4,7,10-Tetraazacyclododecan-1,4,7,10-tetraessigsäure (DOTA), in Betracht.

Gemäß einer weiteren bevorzugten Ausführungsform kann die erfindungsgemäße Paste wenigstens einen Farbstoff beinhalten. Der Farbstoff kann ein fachüblicher Farbstoff und vorzugsweise ein Lebensmittelfarbstoff sein, Ferner kann der Farbstoff in dem wenigstens einen radikalisch polymerisierbaren Monomer löslich oder unlöslich sein. Gemäß einer besonders bevorzugten Ausführungsform wird der Farbstoff aus der Gruppe ausgewählt, die aus E101, E104, E132, E141 (Chlorophyllin), E142, Riboflavin und Lissamingrün besteht. Unter den Begriff Farbstoff fallen erfindungsgemäß auch Farblacke, wie zum Beispiel Farblack Grün, das Aluminiumsalz einer Mischung aus E104 und E132.

Gemäß einer weiteren bevorzugten Ausführungsform kann die erfindungsgemäße Paste wenigstens einen pharmazeutischen Wirkstoff beinhalten. Der wenigstens eine pharmazeutische Wirkstoff kann in der erfindungsgemäßen Paste in gelöster oder suspendierter Form enthalten sein. Der pharmazeutische Wirkstoff kann vorzugsweise aus der Gruppe ausgewählt sein, die aus Antibiotika, Aniphlogistika, Steroiden, Hormonen, Wachstumsfaktoren, Bisphosphonaten, Cytostatika und Genvektoren besteht. Gemäß einer besonders bevorzugten Ausführungsform handelt es sich bei dem wenigstens einen pharmazeutischen Wirkstoff um ein Antibiotikum. Das wenigstens eine Antibiotikum ist vorzugsweise aus der Gruppe ausgewählt, die aus Aminoglykosid-Antibiotika, Glykopeptid-Antibiotika, Lincosamid-Antibiotika, Gyrasehemmem, Carbapenemen, cyclischen Lipopeptiden, Glycylcyclinen, Oxazolidonen und Polypeptidantibiotika besteht Gemäß einer besonders bevorzugten Ausführungsform handelt es sich bei dem wenigstens einen Antibiotikum um ein Mitglied, das aus der Gruppe ausgewählt ist, die aus Gentamicin, Tobramycin, Amikacin, Vancomycin, Teicoplanin, Dalbavancin, Lincosamin, Clindamycin, Moxifloxacin, Levofloxacin, Ofloxacin, Ciprofloxacin, Doripenem, Meropenem, Tigecyclin, Linezolid, Eperezolid, Ramoplanin, Metronidazol, Tinidazol, Omidazol und Colistin sowie Salzen und Estern davon besteht. Demnach kann das wenigstens eine Antibiotikum aus der Gruppe ausgewählt sein, die aus Gentamicinsulfat, Gentamicinhydrochlorid, Amikacinsulfat, Amikacinhydrochlorid, Tobramycinsulfat, Tobramycinhydrochlorid, Clindamycin-hydrochlorid, Lincosaminhydrochlorid und Moxifloxacin besteht. Das wenigstens eine Antiphlogistikum ist vorzugsweise aus der Gruppe ausgewählt, die aus nicht-steroidalen Antiphlogistika und Glukokortikoiden besteht. Gemäß einer besonders bevorzugten Ausführungsform ist das wenigstens eine Antiphlogistikum aus der Gruppe ausgewählt, die aus Acetylsalicylsäure, Ibuprofen, Diclofenac, Ketoprofen, Dexamethason, Prednison, Hydrocortison, Hydrocortisonacetat und Fluticason besteht Das wenigstens eine Hormon ist vorzugsweise aus der Gruppe ausgewählt, die aus Serotonin, Somatotropin, Testosteron und Östrogen besteht. Der wenigstens eine Wachstumsfaktor ist vorzugsweise aus der Gruppe ausgewählt, die aus dem *Fibroblast Growth Factor* (FGF), dem *Transforming Growth Factor* (TGF), dem *Platelet Derived Growth Factor* (PDGF), dem Epidermalen Wachstumsfaktor (EGF), dem *Vascular Endothelial Growth Factor* (VEGF), insulinähnlichen Wachstumsfaktoren (IGF), dem *Hepatocyte Growth Factor* (HGF), dem *Bone Morphogenetic Protein* (BMP), Interleukin-1B, Interleukin 8 und dem Nervenwachstumsfaktor besteht. Das wenigstens eine Cytostatikum ist vorzugsweise aus der Gruppe ausgewählt, die aus Alkylantien, Platinanaloga, Interkalantien, Mitosehemmern, Taxanen, Topoisomerasehemmern und Antimetaboliten besteht. Das wenigstens eine Bisphosphonat ist vorzugsweise aus der Gruppe ausgewählt, die aus Zoledronat und Aledronat besteht.

Gemäß einer weiteren bevorzugten Ausführungsform kann die erfindungsgemäße Paste wenigstens ein biokompatibles Elastomer beinhalten. Das biokompatible Elastomer ist vorzugsweise partikulär. Vorzugsweise ist das biokompatible Elastomer in dem wenigstens einen radikalisch polymerisierbaren Monomer löslich. Als besonders geeignet hat sich die Verwendung von Polybutadien als biokompatibles Elastomer erwiesen.

Gemäß einer weiteren bevorzugten Ausführungsform kann die erfindungsgemäße Paste wenigstens ein Monomer mit Haftgruppen beinhalten. Bei der Haftgruppe kann es sich beispielsweise um eine Amidgruppe handeln. Das Monomer mit Haftgruppe kann daher zum Beispiel Methacrylsäureamid sein. Durch die Verwendung von wenigstens einem Monomer mit Haftgruppen kann die Anbindung des Knochenzementes an Gelenkendoprothesen gezielt beeinflusst werden.

Gemäß einer weiteren bevorzugten Ausführungsform kann die erfindungsgemäße Pasten wenigstens einen Stabilisator beinhalten. Der Stabilisator soll geeignet sein, eine Spontanpolymerisation der in der Paste enthaltenen radikalisch polymerisierbaren Monomere zu verhindern. Ferner soll der Stabilisator keine störenden Wechselwirkungen mit den anderen in der erfindungsgemäßen Paste enthaltenen Bestandteilen zeigten. Derartige Stabilisatoren sind aus dem Stand der Technik bekannt. Gemäß einer bevorzugten Ausführungsform handelt es sich bei dem Stabilisator um 2,6-Di-tert-butyl-4-methylphenol und/oder 2,6-Di-tert-butyl-phenol.

Einen Beitrag zur Lösung der eingangs genannten Aufgabe leistet auch ein Kit, aufweisend eine Paste A und eine Paste B,
wobei
(a) Paste A
   (a1) wenigstens ein radikalisch polymerisierbares Monomer,
   (a2) wenigstens ein in (a1) lösliches Polymer, und
   (a3) wenigstens einen Polymerisationsinitiator beinhaltet;
(b) Paste B
   (b1) wenigstens ein radikalisch polymerisierbares Monomer,
   (b2) wenigstens ein in (b1) lösliches Polymer, und
   (b3) wenigstens einen Polymerisationsbeschleuniger beinhaltet;
   und wobei mindestens eine der Pasten A und B als Komponente (a4) bzw. (b4) wenigstens einen in (a1) bzw. (b1) schwer- oder unlöslichen Füllstoff, beinhaltet, wobei der Füllstoff ein partikuläres, vernetztes Polymethacrylat ist, welches durch Polymerisation von Methacrylsäureestern herstellbar ist, vorzugsweise hergestellt worden ist, wobei

i) mindestens 15 Gew.-% der zur Polymerisation eingesetzten Methacrylsäureester mehrfachfunktionelle Methacrylsäureester sind, bezogen auf das Gesamtgewicht der bei Polymerisation eingesetzten Methacrylsäureester, und
ii) mindestens 90 Gew.-% der Partikel des Füllstoffes, bezogen auf das Gesamtgewicht des Füllstoffes, eine Partikelgröße von nicht mehr als 74 µm aufweisen.

Erfindungsgemäß wird unter Kit ein System aus wenigstens zwei Komponenten verstanden. Obwohl im Folgenden von zwei Komponenten (i. e. Paste A und Paste B) die Rede ist, kann der Kit falls notwendig auch mehr als zwei Komponenten, beispielsweise drei, vier, fünf oder mehr als fünf Komponenten enthalten. Die einzelnen Kitkomponenten liegen vorzugsweise getrennt voneinander verpackt vor, so dass die Inhaltsstoffe der einen Kitkomponente mit den Inhaltsstoffen einer weiteren Kitkomponente nicht in Kontakt stehen. Beispielsweise ist es möglich, die jeweiligen Kitkomponenten getrennt voneinander zu verpacken und zusammen in einem Vorratsbehälter aufzubewahren.

Als radikalisch polymerisierbares Monomer (a1) bzw. (b1), als in (a1) bzw. (b1) lösliches Polymer (a2) bzw. (b2), als Polymerisationsinitiator (a3), als Polymerisationsbeschleuniger (b3) und als partikuläres, vernetztes Polymethacrylat (a4) bzw. (b4) sind diejenigen Komponenten bevorzugt, die bereits eingangs im Zusammenhang mit der erfindungsgemäßen Paste als bevorzugtes radikalisch polymerisierbares Monomer, als in diesem Monomer lösliches Polymer, als Polymerisationsinitiator, als Polymerisationsbeschleuniger und als partikuläres, vernetztes Polymethacrylat beschrieben wurden.

Vorzugsweise beinhalten die Paste A und die Paste B das wenigstens eine radikalisch polymerisierbare Monomer (a1) bzw. (b1) in einer Menge in einem Bereich von 15 bis 85 Gew.-%, mehr bevorzugt 20 bis 70 Gew.-%, noch mehr bevorzugt 25 bis 60 Gew.-% und besonders bevorzugt 25 bis 50 Gew.-%, jeweils bezogen auf das Gesamtgewicht der Paste A bzw. der Paste B.

Vorzugsweise beinhaltet die Paste A den Polymerisationsinitiator (a3) in einer Menge ein einem Bereich von 0,1 bis 10 Gew.-%, mehr bevorzugt in einem Bereich von 0,5 bis 8 Gew.-% und noch mehr bevorzugt in einem Bereich von 1 bis 5 Gew.-%, jeweils bezogen auf das Gesamtgewicht der Paste A.

Vorzugsweise beinhaltet die Paste B den Polymerisationsbeschleuniger (b3) in einer Menge in einem Bereich von 0,0005 bis 0,5 Gew.-%, mehr bevorzugt in einem Bereich von 0,001 bis 0,05 Gew.-% und ganz besonders bevorzugt in einem Bereich von 0,001 bis 0,01 Gew.-%, jeweils bezogen auf das Gesamtgewicht der Paste B.

Sofern eine der beiden Pasten des erfindungsgemäßen Kits den schwer- oder unlöslichen Füllstoff beinhaltet und die andere Paste überhaupt keinen schwer- oder unlöslichen Füllstoff oder einen schwer- oder unlöslichen Füllstoff in einer im Vergleich zur Menge in der anderen Paste vernachlässigbaren Menge, so handelt es sich um einen sogenannten "asymmetrischen" Kit. Ist hingegen der schwer- oder unlösliche Füllstoff in beiden Pasten in etwa vergleichbarer Menge vorhanden, so handelt es sich um einen sogenannten "symmetrischen" Kit.

Weiterhin kann Paste B als weitere Komponente des Initiatorsystems wenigstens ein Alkali- oder Erdalkalihalogenid (b5) beinhalten, wie es in der DE 10 2010 024 653 A1 beschrieben ist, wobei es sich als vorteilhaft erwiesen hat, wenn dieses Alkali- oder Erdalkalihalogenid (b5) in dem radikalisch polymerisierbaren Monomer (b1) löslich ist.

Als Halogenid-Anion kommen grundsätzlich F⁻, Cl⁻ und Br⁻ in Betracht, wobei Cl⁻ ganz besonders bevorzugt ist. Zu den besonders bevorzugten Alkali- oder Erdalkalihalogeniden gehören Kaliumchlorid, Natriumchlorid, Kalziumchlorid und Magnesiumchlorid, wobei Lithiumchlorid als Alkalichlorid (b5) am meisten bevorzugt ist.

In diesem Zusammenhang ist es weiterhin bevorzugt, dass die Paste B das mindestens eine Alkali- oder Erdalkalihalogenid (b5) in einer Menge in einem Bereich von 0,001 bis 7,5 Gew.-%, besonders bevorzugt in einem Bereich von 0,01 bis 5 Gew.%, noch mehr bevorzugt in einem Bereich von 0,1 bis 2,5 Gew.-% und am meisten bevorzugt in einem Bereich von 0,5 bis 1,5 Gew.-%, jeweils bezogen auf das Gesamtgewicht der Paste B, beinhaltet.

Weiterhin können in den Pasten A und/oder B außer den vorstehend beschriebenen Komponenten auch weitere Zusatzstoffe enthalten sein, wie etwa Röntgenopaker, Farbstoffe, pharmazeutische Wirkstoffe, biokompatible Elastomere, Monomere mit Haftgruppen oder Stabilisatoren, wobei auch hier diejenigen Komponenten bevorzugt sind, die bereits eingangs im Zusammenhang mit der erfindungsgemäßen Paste als bevorzugte Röntgenopaker, Farbstoffe, pharmazeutische Wirkstoffe, biokompatible Elastomere, Monomere mit Haftgruppen und Stabilisatoren beschrieben wurden.

Gemäß einer ersten besonderen Ausgestaltung des emndungsgemäßen Kits handelt es sich bei dem Kit um einen "asymmetrischen" Kit. In diesem Zusammenhang ist es bevorzugt, dass die Paste A 20 bis 70 Gew.-%, besonders bevorzugt 25 bis 60 Gew.-%, noch mehr bevorzugt 30 bis 55 Gew.-% und am meisten bevorzugt 34 bis 47 Gew.-%, jeweils bezogen auf das Gesamtgewicht der Paste A, des in (a1) unlöslichen Füllstoffs (a4) und Paste B weniger als 5 Gew.-%, besonders bevorzugt weniger als 1 Gew.-%, noch mehr bevorzugt weniger als 0,1 Gew.-% und darüber hinaus bevorzugt weniger als 0,01 Gew.-%, jeweils bezogen auf das Gesamtgewicht der Paste B, des in (b1) unlöslichen Füllstoffs (b4) beinhaltet, wobei es am meisten bevorzugt ist, dass die Paste B überhaupt keinen in (b1) unlöslichen Füllstoff (b4) beinhaltet.

Weiterhin ist es im Zusammenhang mit dieser ersten besonderen Ausgestaltung des erfindungsgemäßen Kits bevorzugt, dass Paste A das in (a1) lösliches Polymer (a2) in einer Menge in einem Bereich von 1 bis 25 Gew.-%, besonders bevorzugt in einem Bereich von 2 bis 20 Gew.%, noch mehr bevorzugt in einem Bereich von 2 bis 18 Gew.-% und am meisten bevorzugt in einem Bereich von 3 bis 16 Gew.-%, jeweils bezogen auf das Gesamtgewicht der Paste A, und Paste B ein in (b1) lösliches Polymer (b2) in einer Menge in einem Bereich von 25 bis 85 Gew.-%, besonders bevorzugt in einem Bereich von 35 bis 85 Gew.-%, noch mehr bevorzugt in einem Bereich von 40 bis 80 Gew.-% und am meisten bevorzugt in einem Bereich von 50 bis 75 Gew.-%, jeweils bezogen auf das Gesamtgewicht der Paste B, beinhaltet.

Weiterhin ist es im Zusammenhang mit dieser ersten besonderen Ausgestaltung des erfindungsgemäßen Kits bevorzugt, dass das Gewichtsverhälmis von in (b1) unlöslichem Füllstoff (b4) zu dem wenigstens einen in (b1) löslichen Polymer (b2) höchstens 0,2, besonders bevorzugt höchstens 0,15, noch mehr bevorzugt höchstens 0,1, nicht mehr bevorzugt höchstens 0,05, besonders bevorzugt höchstens 0,02 und ganz besonders bevorzugt 0 beträgt.

Gemäß einer zweiten besonderen Ausgestaltung des erfindungsgemäßen Kits handelt es sich bei dem Kit um einen "symmetrischen" Kit. In diesem Zusammenhang ist es bevorzugt, dass die Paste A 15 bis 85 Gew.-%, besonders bevorzugt 15 bis 80 Gew.-% und noch mehr bevorzugt 20 bis 75 Gew.-%, jeweils bezogen auf das Gesamtgewicht der Paste A, des in (a1) unlöslichen Füllstoffs (a4) und Paste B 15 bis 85 Gew.-%, besonders bevorzugt 15 bis 80 Gew.-% und noch mehr bevorzugt 20 bis 75 Gew.-%, jeweils bezogen auf das Gesamtgewicht der Paste B, des in (b1) unlöslichen Füllstoffs (b4) beinhaltet.

Weiterhin ist es im Zusammenhang mit dieser zweiten besonderen Ausgestaltung des erfindungsgemäßen Kits bevorzugt, dass Paste A ein in (a1) lösliches Polymer (a2) in einer Menge in einem Bereich von 5 bis 50 Gew.-%, besonders bevorzugt in einem Bereich von 10 bis 40 Gew.-% und noch mehr bevorzugt in einem Bereich von 20 bis 30 Gew.-%, jeweils bezogen auf das Gesamtgewicht der Paste A, und/oder Paste B ein in (b1) lösliches Polymer (b2) in einer Menge in einem Bereich von 5 bis 50 Gew.-%, besonders bevorzugt in einem Bereich von 10 bis 40 Gew.-% und noch mehr bevorzugt in einem Bereich von 20 bis 30 Gew.-%, bezogen auf das Gesamtgewicht der Paste B, beinhaltet.

Erfindungsgemäß dient die erfindungsgemäße Paste bzw. der erfindungsgemäße Kit, der wenigstens die Pasten A und B enthält, zur Herstellung von Knochenzement.

Im Falle des Kits werden dazu die wenigstens zwei Pasten A und B miteinander vermischt, wobei wiederum eine Paste, Paste C, erhalten wird. Das Mischungsverhältnis beträgt vorzugsweise 0,5 bis 1,5 Gewichtsteile an Paste A und 0,5 bis 1,5 Gewichtsteile an Paste B. Gemäß einer besonders bevorzugten Ausführungsform beträgt der Anteil der Paste A 30 bis 70 Gew.-% und der Anteil der Paste B 30 bis 70 Gew.-%, jeweils bezogen auf das Gesamtgewicht der Pasten A und B. Das Mischen kann mit üblichen Mischgeräten, beispielsweise einem statischen Mischer oder einem dynamischen Mischer erfolgen.

Nach dem Vermischen der Pasten des Kits ist die letztlich erhaltene Paste C nach der Norm ISO 5833 klebfrei und kann sofort verarbeitet werden.

Der aus der erfindungsgemäßen Paste bzw. der aus Paste C durch Aushäitung entstehende Knochenzement erreicht etwa sechs bis acht Minuten nach dem Vermischen der im Kit enthaltenen Pasten eine hohe Festigkeit.

Die erfindungsgemäße Paste bzw. die Paste C, hergestellt aus dem erfindungsgemäßen Kit, kann gemäß einer bevorzugten Ausführungsform zur mechanischen Fixierung von Gelenkendoprothesen, zur Abdeckung von Schädeldefekten, zur Auffüllung von Knochenkavitäten, zur Femuroplastie, zur Vertebroplastie, zur Kyphoplastie, zur Herstellung von Spacern und zur Herstellung von Trägermaterialien für die lokale Antibiotika-Therapie verwendet werden.

Unter dem Begriff *"Spacer"* werden dabei erfindungsgemäß Implantate verstanden, die temporär im Rahmen des zweizeitigen Prothesenwechsels bei septischen Revisionen als Platzhalter verwendet werden.

Trägermaterialien für die lokale Antibiotika-Therapie können als Kugeln oder kugelähnliche Körper oder als bohnenförmige Körper ausgebildet sein. Daneben ist es auch möglich, stabförmige oder scheibenförmige Trägermaterialien herzustellen, die den aus der erfindungsgemäßen Paste bzw. dem erfindungsgemäßen Kit hergestellten Knochenzement hergestellten Knochenzement erhalten. Weiterhin können die Trägermaterialien auch auf resorbierbarem oder nicht resorbierbarem Fadenmaterial perlschnurförmig aufgereiht sein.

Die vorstehend beschriebenen erfindungsgemäßen Verwendungen von Knochenzement sind aus der Literatur bekannt und dort mannigfach beschrieben.

Einen Beitrag zur Lösung der eingangs genannten Aufgaben leistet auch die Verwendung eines in einem radikalisch polymerisierbaren Monomer schwer- oder unlöslichen Füllstoffs, wobei der Füllstoff ein partikuläres, vernetztes Polymethacrylat ist, welches durch Polymerisation von Methacrylsäureestern herstellbar ist, vorzugsweise hergestellt worden ist, wobei
i) mindestens 15 Gew.-% der zur Polymerisation eingesetzten Methacrylsäureester mehrfach funktionelle Methacrylsäureester sind, bezogen auf das Gesamtgewicht der bei Polymerisation eingesetzten Methacrylsäureester, und
ii) mindestens 90 Gew.-% der Partikel des Füllstoffes, bezogen auf das Gesamtgewicht des Füllstoffes, eine Partikelgröße von nicht mehr als 74 µm aufweisen,
als Komponente in einer ein das radikalisch polymerisierbare Monomer als polymerisierbare Komponente beinhaltenden, durch Polymerisation aushärtbaren Zusammensetzung, vorzugsweise in einer durch Polymerisation aushärtbaren Knochenzementpaste.

Als radikalisch polymerisierbares Monomer und als partikuläres, vernetztes Polymethacrylat sind dabei wiederum diejenigen Komponenten bevorzugt, die bereits eingangs im Zusammenhang mit der erfindungsgemäßen Paste als bevorzugtes radikalisch polymerisierbares Monomer und partikuläres, vernetztes Polymethacrylat beschrieben wurden.

Die Erfindung wird durch die nachstehend beschriebenen Beispiel erläutert, die jedoch die Erfindung nicht einschränken.

### AUSFÜHRUNGSBEISPIELE

### Beispiel (erfindungsgemäß)

Es werden Pasten A und B wie im Beispiel 1 der DE 10 2010 024 653 A1 hergestellt. Als nicht lösliches Polymethylmethacrylat wurde jedoch in den Pasten A und B ein Füllstoff eingesetzt, der aus einem Gemisch von 80 Gew-% Methylmethacrylat und 20 Gew-% Ethylenglykoldimethacrylat durch Suspensionspolymerisation unter Verwendung eines thermisch zerfallenden radikalischen Initiators hergestellt wurde. Der Füllstoff hatte eine Korngröße kleiner 63 µm.

Die Pasten A und B wurden nach Lagerung bei Raumtemperatur über Nacht im Gewichtverhältnis 1:1 miteinander gemischt. Es entstanden sofort klebfreie Pasten, die nach wenigen Minuten aushärteten

### Vergleichsbeispiel (nicht erfindungsgemäß)

Es wurden Pasten A und B mit der gleichen Zusammensetzung wie im vorherigen Beispiel hergestellt, wobei jedoch niedriger vernetzte Polymethylmethacrylate als partikulärer Füllstoff verwendet wurden. Ein vernetztes Polymer wurde aus einem Gemisch von 5 Gew-% Ethylenglykoldimethacrylat und 95 Gew-% Methylmethacrylat und ein zweites Polymer aus einem Gemisch von 10 Gew-% Ethylenglykoldimethacrylat und 90 Gew-% Methylmethacrylat synthetisiert. Die mit diesen partikulären Polymeren hergestellten Pasten waren nach Lagerung bei Raumtemperatur über Nacht extrem zäh, lederartig und konnten nicht miteinander zu einem klebfreien Zementteig vermischt werden.

## Patentansprüche

1. Paste, beinhaltend wenigstens ein radikalisch polymerisierbares Monomer, wenigstens ein in dem wenigstens einen radikalisch polymerisierbaren Monomer lösliches Polymer und wenigstens einen in dem wenigstens einen radikalisch polymerisierbaren Monomer schwer- oder unlöslichen Füllstoff, wobei der Füllstoff ein partikuläres, vernetztes Polymethacrylat ist, welches durch Polymerisation von Methacrylsäureestern hergestellt worden ist, wobei
i) mindestens 15 Gew.-% der zur Polymerisation eingesetzten Methacrylsäureester mehrfachfunktionelle Methacrylsäureester sind, bezogen auf das Gesamtgewicht der bei Polymerisation eingesetzten Methacrylsäureester, und
ii) mindestens 90 Gew.-% der Partikel des Füllstoffes, bezogen auf das Gesamtgewicht des Füllstoffes, eine Partikelgröße von nicht mehr als 74 µm aufweisen.

2. Paste nach Anspruch 1, wobei mindestens 20 Gew.-% der zur Polymerisation eingesetzten Methacrylsäureester mehrfachfunktionelle Methacrylsäureester sind, bezogen auf das Gesamtgewicht der bei Polymerisation eingesetzten Methacrylsäureester.

3. Paste nach Anspruch 1, wobei die Paste zwei verschiedene, die Merkmale i) und ii) verwirklichende partilculäre, vernetzte Polymethacrylate P1 und P2 beinhaltet, wobei
- P1 durch Polymerisation von 90 bis 100 Gew.-% mehrfachfunktionellen Methacrylsäureestern und 0 bis 10 Gew.-% monofunktionellen Methacrylsäureestern, jeweils bezogen auf das Gesamtgewicht der bei Polymerisation eingesetzten Methacrylsäureester, erhältlich ist und
- P2 durch Polymerisation von 20 bis 40 Gew.-% mehrfachfunktionellen Methacrylsäureestern und 60 bis 80 Gew.-% monofunktionellen Methacrylsäureestern, jeweils bezogen auf das Gesamtgewicht der bei Polymerisation eingesetzten Methacrylsäureester, erhältlich ist.

4. Paste nach einem der vorhergehenden Ansprüche, wobei der mehrfachfunktionelle Methacrylsäureester ausgewählt ist aus der Gruppe bestehend aus Ethylenglykoldimethacrylat, Butan-1,4-diol-dimethacrylat, Hexan-1,6-diol-dimethacrylat, Dodecan-1,12-diol-dimethacrylat, Diethylen-glykoldimethacrylat, Trimethylenglykoltrimethacrylat, Glycerin-1,2,3-trimethacrylat und Pentaerythroltetramethacrylat.

5. Paste nach einem der vorhergehenden Ansprüche, wobei das radikalisch polymerisierbare Monomer ein Methacrylsäureester ist.

6. Paste nach einem der vorhergehenden Ansprüche, wobei die Paste zusätzlich wenigstens einen Polymerisationsinitiator, wenigstens einen Polymerisationsbeschleuniger oder wenigstens einen Polymerisations-initiator und einen Polymerisationsbeschleuniger beinhaltet.

7. Paste nach einem der vorhergehenden Ansprüche, wobei das lösliche Polymer ausgewählt ist aus der Gruppe bestehend aus Poly(metha-crylsäuremethylester), Poly-(methacrylsäureethylester), Poly-(mettyl-methacrylsäurepropylester), Poly(methacryl-säureisopropylester), Poly(methylmeth-acrylat-co-methyl-acrylat), Poly(styrol-co-methyl-meth-acrylat) und einer Mischung aus mindestens zwei dieser Polymere.

8. Paste nach einem der vorhergehenden Ansprüche, wobei die Paste 15 bis 85 Gew.-%, bezogen auf das Gesamtgewicht der Paste, des in dem mindestens einen radikalisch polymerisierbaren Monomer schwer- oder unlöslichen Füllstoffs beinhaltet.

9. Kit, aufweisend eine Paste A und eine Paste B,
wobei
(a) Paste A
(a1) wenigstens ein radikalisch polymerisierbares Monomer,
(a2) wenigstens ein in (a1) lösliches Polymer, und
(a3) wenigstens einen Polymerisationsinitiator beinhaltet;
(b) Paste B
(b1) wenigstens ein radikalisch polymerisierbares Monomer,
(b2) wenigstens ein in (b1) lösliches Polymer, und
(b3) wenigstens einen Polymerisationsbeschleuniger beinhaltet;
und wobei mindestens eine der Pasten A und B als Komponente (a4) bzw. (b4) wenigstens einen in (a1) bzw. (b1) schwer- oder unlöslichen Füllstoff, wie in einem der Ansprüche 1 bis 5 definiert, beinhaltet.

10. Kit nach Anspruch 9, wobei das radikalisch polymerisierbare Monomer (a1) bzw. (b1) ein Methacrylsäureester ist.

11. Kit nach Anspruch 9 oder 10, wobei der Polymerisationsinitiator (a3) ein Barbitursäurederivat ist.

12. Kit nach Anspruch 11 wobei das Barbitursäurederivat (a3) ausgewählt ist aus der Gruppe bestehend aus 1-monsubstituierten Barbituraten, 5-monosubstituierten Barbituraten, 1,5-disubstituierten Barbituraten- und 1,3,5-trisubstituierten Barbituraten -.

13. Kit nach einem der Ansprüche 9 bis 15, wobei die Paste A den wenigstens einen Polymerisationsinitiator (a3) in einer Menge in einem Bereich von 0,1 bis 10 Gew.-%, bezogen auf das Gesamtgewicht der Paste A, beinhaltet.

14. Kit nach einem der Ansprüche 9 bis 13, wobei der Polymerisationsbeschleuniger (b3) wenigstens eine Schwermetallverbindung ist, die aus der Gruppe bestehend aus Schwermetallsalzen und Schwermetallkomplexen ausgewählt ist.

15. Kit nach Anspruch 14, wobei die Schwermetallverbindung ausgewählt ist aus der Gruppe bestehend aus Kupfer(II)-hydroxid, basischem Kupfer(II)-carbonat, - und einer Mischung aus mindestens zwei davon.

16. Kit nach einem der Ansprüche 9 bis 15, wobei die Paste B den Polymerisationsbeschleuniger (b3) in einer Menge in einem Bereich von 0,0005 bis 0,5 Gew.-%, bezogen auf das Gesamtgewicht der Paste B, beinhaltet.

17. Kit nach einem der Ansprüche 9 bis 16, wobei die Paste A und die Paste B das wenigstens eine radikalisch polymerisierbare Monomer (a1) bzw. (b1) in einer Menge in einem Bereich von 15 bis 85 Gew.-%, jeweils bezogen auf das Gesamtgewicht der Paste A bzw. der Paste B, beinhalten.

18. Kit nach einem der Ansprüche 9 bis 17, wobei das in (a1) bzw. (b1) lösliche Polymer (a3) bzw. (b3) ausgewählt ist aus der Gruppe bestehend aus Poly(methacrylsäuremethylester), Poly(methacrylsäureethylester), Poly-(methylmethacrylsäurepropylester), Poly(methacrylsäureisopropyl-ester), Poly(methylmeth-acrylat-co-methylacrylat), Poly(styrol-co-methyl-meth-acrylat) und einer Mischung aus mindestens zwei dieser Polymere.

19. Kit nach einem der Ansprüche 9 bis 18, wobei Paste A 15 bis 85 Gew.-%, bezogen auf das Gesamtgewicht der Paste A, eines in den Ansprüchen 1 bis 5 definierten Füllstoffs (a4) und Paste B weniger als 5 Gew.-%, bezogen auf das Gesamtgewicht der Paste B, eines in den Ansprüchen 1 bis 5 definierten Füllstoffs (b4) beinhaltet.

20. Kit nach Anspruch 19, wobei Paste A das in (a1) lösliches Polymer (a2) in einer Menge in einem Bereich von 1 bis 25 Gew.-%, bezogen auf das Gesamtgewicht der Paste A, und Paste B das in (b1) lösliches Polymer (b2) in einer Menge in einem Bereich von 25 bis 85 Gew.-%, bezogen auf das Gesamtgewicht der Paste B, beinhaltet.

21. Kit nach einem der Ansprüche 9 bis 18, wobei Paste A 15 bis 85 Gew.-%, bezogen auf das Gesamtgewicht der Paste A, eines in den Ansprüchen 1 bis 5 definierten Füllstoffs (a4) und Paste B 15 bis 85 Gew.-%, bezogen auf das Gesamtgewicht der Paste B, eines in den Ansprüchen 1 bis 5 definierten Füllstoffs (b4) beinhaltet.

22. Kit nach Anspruch 21, wobei Paste A das in (a1) lösliches Polymer (a2) in einer Menge in einem Bereich von 5 bis 50 Gew.-%, bezogen auf das Gesamtgewicht der Paste A, und/oder Paste B das in (b1) lösliches Polymer (b2) in einer Menge in einem Bereich von 5 bis 50 Gew.-%, bezogen auf das Gesamtgewicht der Paste B, beinhaltet.

23. Verwendung eines in einem radikalisch polymerisierbaren Monomer schwer- oder unlöslichen Füllstoffs, wobei der Füllstoff ein partikuläres, vemetztes Polymethacrylat ist, welches durch Polymerisation von Methacrylsäureestern herstellbar ist, wobei
i) mindestens 15 Gew.-% der zur Polymerisation eingesetzten Methacrylsäureester mehrfachfunktionelle Methacrylsäureester sind, bezogen auf das Gesamtgewicht der bei Polymerisation eingesetzten Methacrylsäureester, und
ii) mindestens 90 Gew.-% der Partikel des Füllstoffes, bezogen auf das Gesamtgewicht des Füllstoffes, eine Partikelgröße von nicht mehr als 74 µm aufweisen.
als Komponente in einer das radikalisch polymerisierbare Monomer als polymerisierbare Komponente beinhaltenden, durch Polymerisation aushärtbaren Zusammensetzung.

24. Verwendung einer Paste, wie in einem der Ansprüche 1 bis 8 definiert, oder einer Paste, hergestellt aus dem in den Ansprüchen 9 bis 22 definierten Kit, zur mechanischen Fixierung von Gelenkprothesen, zur Abdeckung von Schädeldefekten, zur Auffüllung von Knochenkavitäten, zur Femuroplastie, zur Vertebroplastie, zur Kyphoplastie, zur Herstellung von Spacern oder zur Herstellung von Trägermaterialien für die lokale Antibiotika-Therapie.
